Europäisches Patentamt
European Patent Office
Office européen des brevets

(19)

(11) Publication number: **0 237 239**
**A2**

(12) **EUROPEAN PATENT APPLICATION**

(21) Application number: 87301739.6

(22) Date of filing: 27.02.87

(51) Int.Cl.[3]: C 07 K 5/06
A 61 K 37/64

(30) Priority: 28.02.86 GB 8605048
16.01.87 GB 8700945

(43) Date of publication of application:
16.09.87 Bulletin 87/38

(84) Designated Contracting States:
AT BE CH DE ES FR GB GR IT LI LU NL SE

(71) Applicant: THE WELLCOME FOUNDATION LIMITED
183-193 Euston Road
London NW1 2BP(GB)

(72) Inventor: Allan, Geoffrey
The Wellcome Foundation Limited Langley Court
Beckenham Kent(GB)

(72) Inventor: Hardy, George William
The Wellcome Foundation Limited Langley Court
Beckenham Kent(GB)

(72) Inventor: Bull, Donald
The Wellcome Foundation Limited Langley Court
Beckenham Kent(GB)

(72) Inventor: O'Callaghan, John Mark
The Wellcome Foundation Limited Temple Hill
Dartford Kent(GB)

(72) Inventor: Lee, Grahame Roy
The Wellcome Foundation Limited Temple Hill
Dartford Kent(GB)

(74) Representative: Garrett, Michael et al,
The Wellcome Foundation Limited Group Patents and
Agreements Langley Court
Beckenham Kent BR3 3BS(GB)

# (54) New compounds.

(57) The present invention is concerned with compounds of formula (I), physiologically acceptable salts thereof, formulations containing them, their preparation and their use in medicine.

In formula (I)

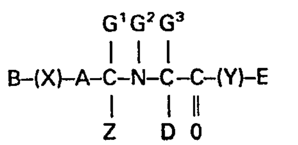

(I)

A is a group of formula $-Q_k-(NQ^1)_m-(CH_2)_n-$, where k and m are independently selected from 0 and 1, provided that m can only be 0 when k is 0, n is from 1 to 6, $Q^1$ is hydrogen or $C_{1-4}$ alkyl, and Q is selected from $-CO-$, $-CH_2-$, $-CH_2CO-$ and $-OCH_2CO-$, and any of the $-(CH_2)_n-$ groups are independently optionally substituted by one or two $C_{1-4}$ alkyl groups;

B is group of formula $-R^1-C(Q^2)(OH)-C(Q^3)(Q^4)-NQ^5-R^2$, where $R^1$ is a bond or $-OC(Q^6)(Q^7)-$, $R^2$ is hydrogen or $C_{1-6}$ alkyl, and $Q^2$–$Q^7$ are independently hydrogen, $C_{1-4}$ alkyl, or a group of formula $-(C(Q^8)(Q^9))_xPh$, in which x is 1 or 2, Ph is a phenyl group optionally substituted by one or more groups independently selected from hydroxy, $C_{1-4}$ alkyl and $C_{1-4}$ alkoxy, and $Q^8$ and $Q^9$ are independently hydrogen or $C_{1-4}$ alkyl;

E and Z are independently selected from carboxyl and carboxyl derivatives;

D is hydrogen or a saturated or unsaturated $C_{1-6}$ aliphatic substituent optionally substituted by an amino group;

$G^1$, $G^2$ and $G^3$ are independently selected from hydrogen and $C_{1-4}$ alkyl; (X) is a mono-, bi-, or tri-cyclic aromatic ring system optionally containing one or more heteroatoms; and

(Y) is a mono- or bi-cyclic nitrogen-containing ring system (attached to $-CO-C(D)(G^3)-$ by said nitrogen atom) optionally containing one or more other heteroatoms and optionally substituted by one or more substituents independently selected from $C_{1-6}$ alkyl and aryl (e.g. phenyl), or (Y) is a nitrogen atom attached to E directly by a bond or indirectly by a $C_{1-4}$ alkylene moiety and also attached to a $C_{5-7}$ cycloalkyl group; in either case (Y) may optionally be linked by a $C_{3-5}$ alkylene bridge to $G^3$ or D to form a closed ring;

and physiologically acceptable salts thereof;

with the proviso that:

*when*, in the definition of A, k and m are both 0 or both 1 and Q is a $-CO-$ group, and, in the definition of B, $R^1$ is a group of formula $-OC(Q^6)(Q^7)-$ as hereinbefore defined and $R^2$ is a $C_{1-6}$ alkyl group;

D is hydrogen or a $C_{1-6}$ alkyl group;

(X) is a benzene ring or a naphthyl or indolyl ring system, any of which is optionally substituted in any position by one or more substituents independently selected from $C_{1-4}$ alkyl (itself optionally substituted by one or more halogen atoms), $C_{1-4}$ alkoxy, halo, nitro, amino, carboxyl, $C_{1-4}$ alkoxycarbonyl and hydroxy,

*then* (Y) is not a pyrrolidinyl, oxazolidinyl, or thiazolidinyl ring, or a ring system selected from indolinyl, quinolinyl and tetrahydroquinolinyl.

EP 0 237 239 A2

0237239

# NEW COMPOUNDS

This invention relates to new chemical compounds, their preparation, compositions containing them and their use in medicine.

Compounds which exhibit β-andrenergic blocking activity, for example, as described in U.S. Patent 3,705,907, are widely used in the therapy of hypertensive disorders. Another class of agents used for the treatment of hypertension are inhibitors of angiotensin converting enzyme (ACE), for example, as described in European Patent 0012401 A1. It is now believed that compounds which are simultaneously both β-blockers and ACE inhibitors are especially useful in the treatment and/or prophylaxis of hypertension.

We have now found that compounds of formula (I) (as defined hereinbelow) and their physiologically acceptable salts exhibit both β-blockade and ACE inhibitory activity.

In formula (I)

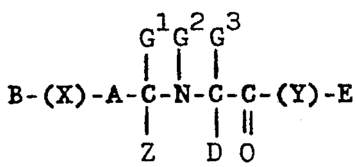

(I)

A is a group of formula $-Q_k-(NQ^1)_m-(CH_2)_n-$, where k and m are independently selected from 0 and 1, provided that m can only be 0 when k is 0, n is from 1 to 6, $Q^1$ is hydrogen or $C_{1-4}$ alkyl, Q is selected from -CO-, $-CH_2-$, $-CH_2CO-$ and $-OCH_2CO-$, and any of the $-(CH_2)_n-$ groups are independently optionally substituted by one or two $C_{1-4}$ alkyl groups;

B is group of formula $-R^1-CQ^2(OH)-C(Q^3)(Q^4)-NQ^5-R^2$, where $R^1$ is a bond or $-OC(Q^6)(Q^7)-$, $R^2$ is hydrogen or $C_{1-6}$ alkyl, and $Q^2$-$Q^7$ are independently hydrogen, $C_{1-4}$ alkyl, or a group of formula $-(C(Q^8)(Q^9))_xPh$, in which x is 1 or 2, Ph is a phenyl group optionally substituted by one or more groups independently selected from hydroxy, $C_{1-4}$ alkyl and $C_{1-4}$ alkoxy, and $Q^8$ and $Q^9$ are independently hydrogen or $C_{1-4}$ alkyl;

E and Z are independently selected from carboxyl and carboxyl derivatives, e.g. esters;

D is hydrogen or a saturated or unsaturated $C_{1-6}$ aliphatic substituent optionally substituted by an amino group;

$G^1$, $G^2$ and $G^3$ are independently selected from hydrogen and $C_{1-4}$ alkyl;

(X) is a mono-, bi-, or tri-cyclic aromatic ring system optionally containing one or more heteroatoms; and

(Y) is a mono- or bi-cyclic nitrogen-containing ring system (attached to $-CO-C(D)(G^3)-$ by said nitrogen atom) optionally containing one or more other heteroatoms and optionally substituted by one or more substituents independently selected from $C_{1-6}$ alkyl and aryl (e.g. phenyl), or (Y) is a nitrogen atom attached to E directly by a bond or indirectly by a $C_{1-4}$ alkylene moiety and also attached to a $C_{5-7}$ cycloalkyl group; in either case (Y) may optionally be linked by a $C_{3-5}$ alkylene bridge to $G^3$ or D to form a closed ring;

and physiologically acceptable salts thereof;

with the proviso that:

<u>when</u>, in the definition of A, k and m are both 0 or both 1 and Q is a -CO-group, and, in the definition of B, $R^1$ is a group of formula $-OC(Q^6)(Q^7)-$ as hereinbefore defined and $R^2$ is a $C_{1-6}$ alkyl group;

D is hydrogen or a $C_{1-6}$ alkyl group;

(X) is a benzene ring or a naphthyl or indolyl ring system, any of which is optionally substituted in any position by one or more substituents independently selected from $C_{1-4}$ alkyl (itself optionally substituted by one or more halogen atoms), $C_{1-4}$ alkoxy, halo, nitro, amino, carboxyl, $C_{1-4}$ alkoxycarbonyl and hydroxy,

then (Y) is not a pyrrolidinyl, oxazolidinyl, or thiazolidinyl ring, or a ring system selected from indolinyl, quinolinyl and tetrahydroquinolinyl.

Preferably, in the definition of A, n is 3-5, especially 4. In the definition of B, $R^2$ is preferably a $C_{1-4}$ alkyl group, that is a methyl, ethyl, n-propyl, iso-propyl, n-butyl, iso-butyl, or t-butyl group; iso-propyl is particularly preferred. Carboxyl derivatives in the definitions of E and Z include esters, particularly benzyl and $C_{1-4}$ alkyl esters, hydroxymethylene and carbamoyl. Most preferably, D is a $C_{1-4}$ alkyl group, especially a methyl group.

The ring system denoted by (X) may, for example, have the formula:

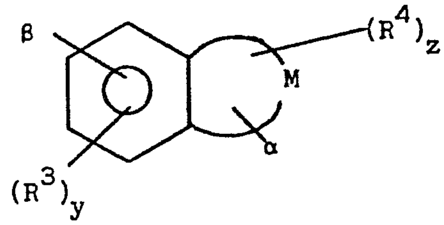

where β is the point of attachment of B, α is the point of attachment of A, 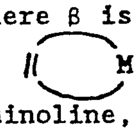 M is a pyrrole, pyridine, or benzene ring (i.e. (X) is an indole, quinoline, or naphthalene ring system respectively), y and z are independent integers of from 0 to 3, and $R^3$ and $R^4$ are independently selected from halo, $C_{1-4}$ alkyl (which may itself be substituted by one or more halo groups), and hydroxy. The ring system denoted by (X) may also, for example, be a benzene ring, optionally substituted by one or more groups as defined for $R^3$ and $R^4$ above. Another particularly preferred ring system for (X) is carbazole, especially when A and B are each attached to a different ring thereof, the two phenyl rings being optionally substituted by groups $(R^3)_y$ and $(R^4)_z$ respectively as hereinbefore defined.

The ring system denoted by (Y) may be selected from pyrrolyl, imidazolyl, pyrazolyl, pyridyl, pyrazinyl, pyrimidinyl, pyridazinyl, pyrrolidinyl, pyrrolinyl, imidazolidinyl, imidazolinyl, pyrazolidinyl, pyrazolinyl, piperidinyl, piperizinyl, 1H- indolyl, 3H-indolyl, oxazolidinyl,-quinolyl, tetrahydroquinolyl, and morpholino, or, in general, may be a group selected from those of formula:

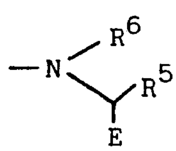

where (i) $R^5$ and $R^6$ may together form a $C_{2-5}$ alkylene moiety to give a nitrogen-containing ring, which itself may be fused with a $C_{4-7}$ cycloalkyl ring or with a $C_{5-7}$ aryl ring (e.g. phenyl); or

(ii) $R^5$ may be hydrogen or $C_{1-4}$ alkyl and $R^6$ a $C_{5-7}$ cycloalkyl group;

As indicated earlier, in either case, (i) or (ii), $R^5$ may be optionally linked with the group D or $G^3$ in formula (I) by a $C_{3-5}$ alkylene bridge to form a closed ring.

Suitable groups for (Y) may be any of those shown as the N-terminal group of the ACE inhibitors described in J. Cardiovasc. Pharmacol., Vol. 7 (Suppl. 1), 1985, p. S4 (H.R. Brunmer et al) or in European Patent 0159156 A1.

Preferred compounds according to the present invention are:

N-(1-Carboxy-5-[5-(2-hydroxy-3-isopropylaminopropoxy)carbazole-3-carboxamido]pentyl)alanylproline,

N-(1-Carboxy-5-[5-(2-hydroxy-3-isopropylaminopropoxy)carbazole-2-carboxamido]pentyl)alanylproline,

N-(1-Carboxy-5-[8-(2-hydroxy-3-isopropylaminopropoxy)naphth-2-ylmethylamino]pentyl)alanylproline,

N-(1-Carboxy-5-[5-(2-hydroxy-3-isopropylaminopropoxy)naphth-2-ylmethylamino]pentyl)alanylproline,

N-(1-Carboxy-5-[4-(2-hydroxy-3-isopropylaminopropoxy)-1H-indol-2-ylmethylamino]pentyl)alanylproline,

N-(1-Carboxy-5-[4-(2-hydroxy-3-isopropylaminopropoxy)-1H-indol-3-ylmethylamino]pentyl)alanylproline,

N-(1-Carboxy-5-[8-(2-hydroxy-3-isopropylaminopropoxy)quinoline-2-carboxamido]pentyl)alanylproline,

N-(1-Carboxy-5-[5-(2-hydroxy-3-isopropylaminopropoxy)quinoline-2-carboxamido]pentyl)alanylproline,

N-(1-Carboxy-5-[4-(2-hydroxy-3-isopropylaminopropoxy)benzylcarboxamido]-pentyl)alanylproline, and

N-(1-Carboxy-5-(2-[5-(2-hydroxy-3-isopropylaminopropoxy)naphth-1-yloxy]acetamido)pentyl]alanylproline,

and their half esters (wherein Z is $C_{2-5}$ carboalkoxy),

in any of their individual isomeric forms, and salts thereof.

Particularly preferred isomers of the above compounds are:

N-(1(S,R)-Carboxy-5-[5-(2(S,R)-hydroxy-3-isopropylaminopropoxy)carbazole-3-carboxamido]pentyl)-(S)-alanyl-(S)-proline,

N-(1(S,R)-Carboxy-5-[5-(2(S,R)-hydroxy-3-isopropylaminopropoxy)carbazole-2-carboxamido]pentyl)-(S)-alanyl-(S)-proline,

N-(1(S)-Carboxy-5-[5-(2(S,R)-hydroxy-3-isopropylaminopropoxy)carbazole-3-carboxamido]pentyl)-(S)-alanyl-(S)-proline,

N-(1(S)-Carboxy-5-[5-(2(S,R)-hydroxy-3-isopropylaminopropoxy)carbazole-2-carboxamido]pentyl)-(S)-alanyl-(S)-proline,

N-(1(S)-Carboxy-5-(8-(2(S,R)-hydroxy-3-isopropylaminopropoxy)naphth-2-ylmethylamino]pentyl)-(S,R)-alanyl-(S)-proline,

N-(1(S)-Carboxy-5-[5-(2(S,R)-hydroxy-3-isopropylaminopropoxy)naphth-2-ylmethylamino]pentyl)-(S)-alanyl-(S)-proline,

N-(1(S)-Carboxy-5-[4-(2(S,R)-hydroxy-3-isopropylaminopropoxy)-1H-indol-2-ylmethylamino]pentyl)-(S)-alanyl-(S)-proline,

N-(1(S)-Carboxy-5-[4-(2(S,R)-hydroxy-3-isopropylaminopropoxy)-1H-indol-3-ylmethylamino]pentyl)-(S)-alanyl-(S)-proline,

N-(1(S)-Carboxy-5-[8-(2(S,R)-hydroxy-3-isopropylaminopropoxy)quinoline-2-carboxamido]pentyl)-(S)-alanyl-(S)-proline,

N-(1(S)-Carboxy-5-[5-(2(S,R)-hydroxy-3-isopropylaminopropoxy)quinoline-2-carboxamido]pentyl)-(S)-alanyl-(S)-proline,

N-(1(S)-Carboxy-5-[4-(2(S,R)-hydroxy-3-isopropylaminopropoxy)benzylcarboxamido]pentyl)-(S,R)-alanyl-(S)-proline, and

N-(1(S)-Carboxy-5-(2-[5-(2(S,R)-hydroxy-3-isopropylaminopropoxy)naphth-1-yloxy]acetamido)pentyl]-(S,R)-alanyl-(S)-proline.

Of these, N-(1(S)-carboxy-5-(8-(2(S,R)-hydroxy-3-isopropylaminopropoxy) naphth-2-ylmethylamino]pentyl)-(S,R)-alanyl-(S)-proline, its half esters (as defined above), and their physiologically acceptable salts are particularly preferred by virtue of their especially advantageous combination of ACE-inhibitory and β-blocking properties.

This preferred aspect of the invention also extends to the half esters (as defined above) and to the physiologically acceptable salts of said compounds and to any mixture of two or more individual isomers of one or more of the particular compounds referred to above.

The physiologically acceptable salts of compounds of formula (I) include salts derived from organic and inorganic acids as well as from bases. Suitable salts derived from acids include, for example, the acetate, adipate, alginate, aspartate, benzoate, benzenesulphonate, bisulphate, butyrate, citrate, camphorate, camphorsulphonate, cyclopentanepropionate, digluconate, dodecylsulphate, ethanesulphonate, fumarate, glucoheptanoate, glycerophosphate, hemisulphate, heptanoate, hexanoate, hydrochloride,

glycerophosphate, hemisulphate, heptanoate, hexanoate, hydrochloride, hydrobromide, hydroiodide, 2-hydroxyethanesulphonate, lactate, maleate, methanesulphonate, 2-naphthalenesulphonate, nicotinate, oxalate, palmoate, pectinate, persulphate, 3-phenylpropionate, picrate, pivalate, propionate, succinate, tartrate, thiocyanate, tosylate, and undecanoate.

Base salts include ammonium salts, alkali metal salts such as those of sodium and potassium, alkaline earth metal salts such as those of calcium and magnesium, salts with organic bases such as dicyclohexylamine and N-methyl-D-glucamine, and salts with amino acids such as arginine and lysine.

Quaternary ammonium salts can be formed, for example, by reaction with lower alkyl halides, such as methyl, ethyl, propyl, and butyl chlorides, bromides and iodides, with dialkyl sulphates, with long chain halides, such as decyl, lauryl, myristyl and stearyl chlorides, bromides and iodides, and with aralkyl halides, such as benzyl and phenethyl bromides.

In another aspect, the present invention provides compounds of formula (I) and physiologically acceptable salts thereof for use in medicine, and in particular in a method of prophylaxis or treatment of the human or animal body, by therapy, or of diagnosis. The compounds and salts may be used wherever an ACE inhibitor and/or $\beta$-blocker is indicated. Thus, for example, the compounds of formula (I) and their physiologically acceptable salts may be used for the treatment of all forms of hypertension in both the long and the short term (particularly those forms associated with high serum renin levels) and, in general, for the treatment of heart failure. Compounds of formula (I) and their physiologically acceptable salts may also be used in the treatment of hyperaldosteronism.

The amount of a compound of formula (I) or a physiologically acceptable salt thereof which is required to achieve the desired biological effect will, of course, depend on a number of factors, for example, the specific compound chosen, the use for which it is intended, the mode of administration, and the recipient. In general, a daily dose is expected to lie in the range of from 1ng to 100mg, typically from 50ng to 50mg, per day

per kilogram bodyweight, for example 500ng-5mg/kg/day. An intravenous dose may, for example, be in the range of from 10ng to 1 mg/kg which may conveniently be administered as an infusion of from 0.1ng to 50μg per kilogram per minute. Infusion fluids suitable for this purpose may contain, for example, from 0.01ng to 100μg, typically from 0.1ng to 100μg, per millilitre. Unit doses, for example, may contain from 100ng to 100mg of the active compound; for example, ampoules for injection may contain from 100ng to 1mg and orally administrable unit dose formulations, such as tablets or capsules, may contain, for example, from 0.001 to 50mg, typically from 0.02 to 20mg. In the case of physiologically acceptable salts, the weights indicated above refer to the weight of the active compound ion, that is the ion derived from the compound of formula (I).

In the treatment or prophylaxis of the conditions referred to above, while the compounds of formula (I) and their physiologically acceptable salts may be used as the compound per se, but they are preferably presented with an acceptable carrier therefor as a pharmaceutical formulation in accordance with the present invention. The carrier must, of course, be acceptable in the sense of being compatible with the other ingredients of the formulation and not be deleterious to the recipient thereof. The carrier may be a solid or a liquid, or both, and is preferably formulated with the compound as a unit-dose formulation, for example, a tablet, which may contain from 0.05% to 95% by weight of active compound. Other pharmacologically active substances may also be present in formulations of the present invention. One or more of the compounds of formula (I) and/or their physiologically acceptable salts may be incorporated in the formulations. The formulations may be prepared by any of the well known techniques of pharmacy consisting essentially of admixture of the components.

The formulations include those suitable for oral, rectal, topical buccal (e.g. sub-lingual), and parenteral (e.g. subcutaneous, intramuscular, intradermal, or intravenous) administration, although the most suitable route in any given case will depend on the nature and severity of the condition being treated and on the nature of the particular compound of formula (I), or the physiologically acceptable salt thereof, which is being used.

Formulations suitable for oral administration may be presented in discrete units, such as capsules, cachets, lozenges or tablets, each containing a predetermined amount of a compound of formula (I) or a physiologically acceptable salt thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and the carrier (which may constitute one or more accessory ingredients). In general, the formulations are prepared by uniformly and intimately admixing the active compound with a liquid or finely divided solid carrier or both, and then, if necessary, shaping the product. For example, a tablet may be prepared by compressing or moulding a powder or granules of the compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Moulded tablets may be made by moulding, in a suitable machine, the powdered compound moistened with an inert liquid diluent.

Formulations suitable for buccal (sub-lingual) administration include lozenges comprising a compound of formula (I), or a physiologically acceptable salt thereof, in a flavoured base, usually sucrose and acacia or tragacanth; and pastilles comprising the compound in an inert base such as gelatin and glycerin or sucrose and acacia.

Formulations of the present invention suitable for parenteral administration conveniently comprise sterile aqueous preparations of a compound of formula (I), or a physiologically acceptable salt thereof, which preparations are preferably isotonic with the blood of the intended recipient. These preparations are preferably administered intravenously, although administration may also be effected by means of subcutaneous, intramuscular, or intradermal injection. Such preparations may conveniently be prepared by admixing the compound with water and rendering the resulting solution sterile and isotonic with the blood. Injectable compositions

according to the invention will generally contain from 0.1 to 5% w/w of active compound.

Formulations suitable for rectal administration are preferably presented as unit-dose suppositories. These may be prepared by admixing a compound of formula (I), or a physiologically acceptable salt thereof, with one or more conventional solid carriers, for example, cocoa butter, and then shaping the resulting mixture. Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. The active compound is generally present at a concentration of from 0.1 to 15% w/w of the composition, for example, from 0.5 to 2%.

The compounds of formula (I) and their physiologically acceptable salts may be prepared in any conventional manner and in accordance with the present invention, may for example be prepared by any method hereinafter described. In particular, the compounds of formula (I) and their physiologically acceptable salts may be prepared by any of the methods standard in the art of peptide chemistry and may comprise coupling the aryl moiety, the amino-(hydroxy)alkoxy side chain and the amino acids or derivatives thereof which together form the compounds of formula (I), in any desired order. It will be appreciated that for example, the aryl moiety, side chain and the amino acids or their derivatives may be coupled in any order to form two separate chemical entities which, as a final process stage (which we may claim as an aspect of the present invention) are then coupled to form the compound of formula (I) or a precursor therefor and if appropriate, converting the precursor (which conversion we may also claim as an aspect of the present invention) to the desired compound of formula (I), and optionally if desired, converting the compound of formula (I) to another compound of formula (I), and also optionally if desired, converting any compound of formula (I) so formed to a physiologically acceptable salt thereof.

Thus, according to the present invention, we also provide a process for the preparation of a compound of formula (I) or a physiologically acceptable

salt thereof which comprises reacting a compound $R_1$ with a compound $R_2$, wherein:

(a) $R_1$ is a compound of formula (II):

$$B-(X)-Q-R^7 \qquad (II)$$

and $R_2$ is a compound of formula (III):

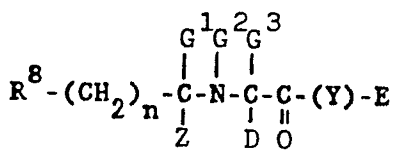

(III)

;or

(b) $R_1$ is a compound of formula (IV):

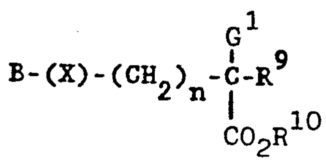

(IV)

and $R_2$ is a compound of formula (V):

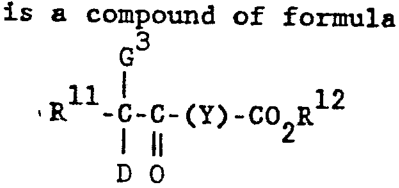

(V)

;or

(c) $R_1$ is a compound of formula (VI):

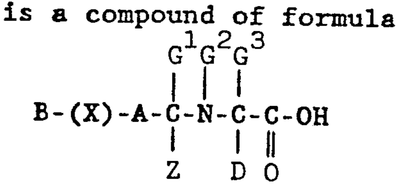

(VI)

and $R_2$ is a compound of formula (VII):

$$H-(Y)-E \qquad (VII)$$

wherein the hydrogen H is bonded to the nitrogen atom of (Y).

In the compounds of formulae (II) to (VII), A, (B), D,E, $G^1$ to $G^3$, n, X, (Y) and Z are as hereinbefore defined, $R^7$, $R^8$, $R^9$ and $R^{11}$ are independently selected from an amino group, an appropriate derivative thereof, or a leaving group such that when $R^7$ and/or $R^9$ is an amino group or an appropriate derivative thereof, $R^8$ and/or $R^{11}$ is a leaving group, or, conversely, when $R^8$ and/or $R^{11}$ is an amino group or an appropriate derivative thereof, $R^7$ and/or $R^9$ is a leaving group, and $R^{10}$ and $R^{12}$ are suitable carboxyl protecting groups;

and subsequently if desired, performing one or more of the following optional reactions in any desired order:-

(i) where the product of the reaction of compounds $R_1$ and $R_2$ is a precursor to a compound of formula (I) subjecting that precursor to such conditions and/or treating it with an appropriate agent or agents to bring about formation of a compound of formula (I);

(ii) converting any compound of formula (I) so formed to another compound of formula (I); and

(iii) converting any compound of formula (I) to a physiologically acceptable salt thereof.

In formulae (III) and (VII) above and throughout this specification, any of the $-(CH_2)_n-$ groups forming part of a formula of an intermediate (e.g. as occuring herein in formulae (III), (IV), and (XV)) are each independently optionally substituted by one or two $C_{1-4}$ alkyl groups.

The above optional reactions (i) - (iii) constitute further aspects of the present invention and may be claimed herein individually or in any desired combination.

WMD/OLM/29th January 1987

or more moieties thereof are protected with a suitable protecting group, and the reaction comprises subjecting the precursor to such conditions and/or treating it with an agent or agents thereby to bring about deprotection with consequent formation of the desired compound of formula (I).

Details of the methods of peptide chemistry, and in particular suitable activating and protecting groups and of suitable reaction conditions for the above processes may be found in the following literature which is given purely by way of exemplification and is not intended to be either exhaustive or limiting:

a) Schroder and Luebke, "The Peptides" (Academic Press) (1965).

b) Bellean and Malek, J.Am.Chem. Soc., 90, 165, (1968).

c) Tilak, Tetrahedron Letters, 849 (1970).

d) Beyerman, Helv. Chim. Acta., 56, 1729 (1973).

e) Stewart and Young, "Solid Phase Peptide Synthesis" (W.H.Freeman and Co.) (1969).

f) "Methoden der Organischen Chemie" (Houben-Weyl), 4th Edn., Vol.15, "Synthese von Peptiden", Parts 1 and 2 (Georg Thieme Verlag, 1974).

g) "The Peptides: Analysis, Synthesis, Biology", Gross, E. and Meienhofer, J. eds., Vols. 1 to 4 (Academic Press, 1979).

h) "Peptides: Syntheses, Physical Data", Voelter, W. and Schmid-Siegmann, E., Vols. 1 to 6 (George Thieme Verlag, 1983).

i) E. Atherton, M. J. Gait, R. C. Sheppard and B. J. Williams, Bioorganic Chem., 1979, 8, 351-370.

j) R. C. Sheppard, Chemistry in Britain, 1983, 402-414.

k) E. Atherton, E. Brown and R. C. Sheppard, J.C.S. Chem. Comm. 1981, 1151-1152.

l) T.W. Greene, Protective Groups in Organic Synthesis, Wiley, New York, 1981.

m) G.C. Barrett (Ed.), The Chemistry and Biochemistry of the Amino Acids, 1st Edn., Chapman and Hall, London and New York, 1985.

As usual in peptide chemistry, different protecting groups may be used simultaneously on different moieties of a molecule to enable selective deprotection at different reaction stages, according to the reagents and reaction conditions in use. Thus for example, the protecting groups BOC (t-butoxycarbonyl) and TBDMS (t-butyldimethylsilyl) may be removed by treatment with a strong acid such as hydrochloric acid, preferably in an appropriate solvent such as methanol. The protecting group Z (benzyloxycarbonyl) may be removed by hydrogenation over palladium-carbon, preferably under acidic conditions, in a suitable solvent such as methanol. When it is necessary to protect a carboxy group such as defined for the groups E and Z above, this may conveniently be effected by formation of a corresponding simple ester such as the t-butyl ester and subsequent deprotection may be performed by acid hydrolysis.

Optional reaction (i) also includes the case wherein, in the precursor to the compound of formula (I), B represents a reactive substituent R, or protected form thereof, on group (X), and the reaction comprises treating that product with an agent or agents serving to effect substitution of the group B onto (X).

In that case, for the preparation of compounds of formula (I) wherein $Q^5$ is hydrogen, the reactive substituent R may be hydroxy and the precursor reacted with a compound of formula (XXIX):

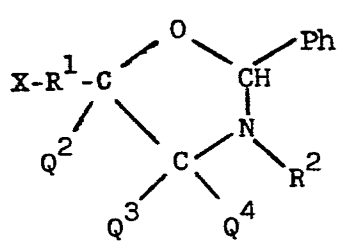

(XXIX)

wherein $Q^2$ to $Q^4$, $R^1$ and $R^2$ are as hereinbefore defined, Ph is phenyl and X is an appropriate leaving group such as mesyl, and the reaction is conveniently effected under basic conditions, followed by

deprotection/decyclisation with an appropriate reagent such as trifluoroacetic acid.

Alternatively, the reactive substituent R may be a group of formula (XXX):

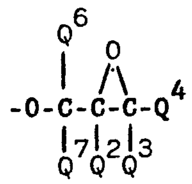

(XXX)

wherein $Q^2$ to $Q^4$, $Q^6$ and $Q^7$ are as hereinbefore defined, and the reagent is an appropriate amine, the reaction conveniently being performed by heating in an appropriate solvent such as dioxan.

It will be appreciated that instead of substituting the group B onto the ring or ring system (X) as part of optional reaction (i), as an aspect of the present invention, an equivalent subsitution may be made at any appropriate stage during the entire procedure for synthesis of the compounds of formula (I) and their physiologically acceptable salts. In particular, this substitution may be effected by following either of the procedures specified above in respect of the compounds of formulae (XXIX) and (XXX) but then in place of the precursor to the compound of formula (I) in which R represents a reactive substituent, or protected form thereof, using an appropriate intermediate including said group R, when R represents a reactive substituent or protected form thereof. Examples of intermediates suitable for this purpose are those defined herein by the formulae (II), (IV), (VI), (VIII), (IX), (X), (XI), (XII), (XIII), (XIV), (XVI), and (XXVIII). Such procedures may, as appropriate, optionally require one or more steps of protection and/or deprotection as required.

Optional reaction (i) further includes the case wherein a compound of formula (I) wherein Z is an ester is formed by reaction of the corresponding compound of formula (I) wherein Z is carbamoyl with the appropriate alcohol, in the presence of an acid catalyst, preferably of the ion exchange type, such as Amberlyst 15.

Optional reaction (ii) may comprise the case wherein in the compound of formula (I), E and/or Z are/is a carboxyl group, converting the compound to a corresponding compound wherein E and/or Z are/is a carboxylic acid derivative, for example by treatment of the compound with an appropriate alcohol in the presence of a suitable acid such as sulphuric acid.

Optional reaction (ii) may also comprise the case wherein in the compound of formula (I), E and/or Z are/is a carboxylic acid derivative, converting the compound to a corresponding compound wherein E and/or Z are/is a carboxyl group, for example by hydrolysis, e.g. under basic conditions, for example in the presence of sodium hydroxide.

Optional reaction (iii) may comprise reacting the compound of formula (I) thus formed, with an appropriate mineral or organic acid.

Specific sub-classes of the above process according to the invention include the following:

(A) As a preferred sub-class of reaction variant (a), compounds of formula (I) wherein k is 1 and Q is other than methylene may be prepared by reacting a compound of formula (II) wherein B, Q, and (X) are as hereinbefore defined, except that Q is not methylene, and $R^7$ is a hydroxy group and/or together with Q forms a carboxyl group or a derivative thereof, with a compound of formula (III) wherein D,E,$G^1$ to $G^3$, n, and Z are as hereinbefore defined and $R^8$ is an amino group or derivative thereof.

(B) As a preferred sub-class of optional reaction (i), compounds of formula (I) may be prepared by reducing a compound of formula (VIII), (IX), or (X):

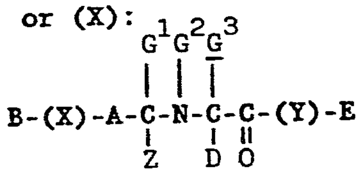

(VIII)

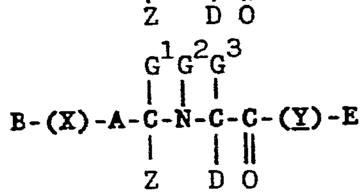

(IX)

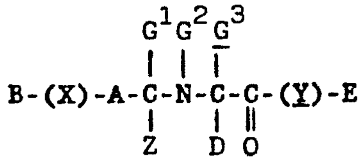

(X)

wherein A,B,D,E,$G^1$ to $G^3$, (X), (Y), and Z are as hereinbefore defined, or, as appropriate, protected forms thereof, and $\underline{G}^3$ and ($\underline{Y}$) are unsaturated forms of $G^3$ and (Y) respectively.

(C) As a further preferred sub-class of optional reaction (i), compounds of formula (I) wherein $G^1$ and $G^2$ are hydrogen or $G^3$ is hydrogen may be prepared by reducing a compound of formula (XI) or (XII) respectively:

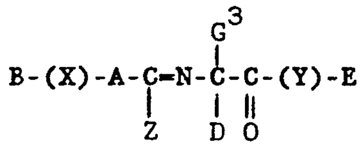

(XI)

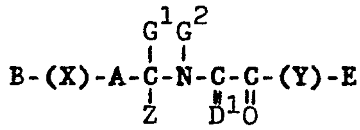

(XII)

wherein A,B,D,E,$G^1$ to $G^3$, (X), (Y) and Z are as hereinbefore defined, or, as appropriate, protected forms thereof, and $D^1$ is a group analogous to D as hereinbefore defined (other than hydrogen) which lacks one hydrogen atom so that it is bonded to the carbon of the -N($G^2$)-C- moiety by a double bond.

(D) As a further preferred sub-class of optional reaction (i) compounds of formula (I) wherein Z is carboxyl or carbamoyl may be prepared by treating a compound of formula (XIII):

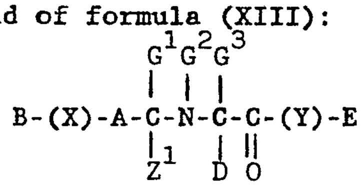

(XIII)

wherein A,B,D,E,$G^1$ to $G^3$, (X) and (Y) are as hereinbefore defined, or,as appropriate, protected forms thereof, and $Z^1$ is an appropriate carboxyl or carbamoyl precursor group, with an agent or agents capable of converting the precursor group $Z^1$ to a carboxyl or carbamoyl group;

and, if desired, effecting one or more of the optional reactions (i) to (iii) referred to above, or, as appropriate, one or more further of said optional reactions, in any desired order.

Reaction (a), including reaction A, is conveniently performed using dicyclohexylcarbodiimide (DCCI) in the presence of 1-hydroxybenzotriazole (HOBT) to suppress recemisation, in a suitable solvent such as methylene chloride or DMF at a reduced temperature, e.g. in the range -15°C to 0°C, or at room temperature, as appropriate. Such a reaction protocol is hereinafter termed 'DCCI/HOBT' conditions.

Reaction (b) is conveniently performed at room temperature or with heating, as appropriate, in a suitable solvent such as dichloromethane or DMF.

Reaction (c) is conveniently performed under DCCI/HOBT conditions, or by mixed anhydride coupling, which is a technique well known to those skilled in the art of peptide chemistry, but by way of example, may comprise reacting the acid intermediate with isobutylchloroformate and N-methylmorpholine, followed by reaction with the amine intermediate, the reaction conveniently being performed at a reduced temperatue, e.g. from -15°C to -25°C, to minimise racemisation.

Reaction (B) of optional reaction (i) is conveniently performed by hydrogenation over a suitable catalyst, such as palladium on carbon, for example in a suitable solvent such as methanol. Preferred classes of compounds of formulae (IX) and (X) are those wherein -(Y)-E is 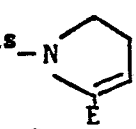 .

Reaction (C) is also conveniently performed by hydrogenation, for example, by the method described in the preceeding paragraph. It will be appreciated that in this reaction, the intermediate of formula (XI) or (XII) may be forred *in situ* (for example, by a method as described hereinbelow) and that a compound of formula (XI) wherein $G^3$ is hydrogen can also be represented by the resonance form (XIA):

```
           H
           |
B-(X)-A-C-N=C-C-(Y)-E                    (XIA)
           |     |  ||
           Z     D  O
```

wherein A,B,D,E,(X),(Y), and Z are as hereinbefore defined. A preferred class of compounds of formula (XII) are those wherein $D^1$ is methylene.

Again it will appreciated that a compound of formula (XII) wherein $G^2$ is hydrogen can also be represented by the resonance form (XIIA):

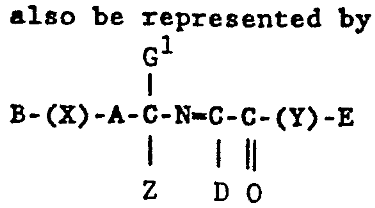

(XIIA)

wherein A,B,D,E,(X),(Y) and Z are as hereinbefore defined.

Reaction (D) may, for example, comprise hydrolysis of a compound of formula (XIII) in which $Z^1$ represents cyano. Such hydrolysis is conveniently performed by acid hydrolysis, eg in a suitable strong mineral acid such as hydrochloric acid, in a suitable solvent, such as methanol. Alternatively, reaction (D) may comprise reacting a compound of formula (XIII) wherein $Z^1$ is an appropriate carbonyl derivative group with one or more suitable reagents capable of aminating said group. For example, when $Z^1$ is an activated carbonyl group such as an ester or an acid chloride, the compound of formula (XIII) may be reacted with ammonia, preferably under aqueous conditions. If the carbonyl derivative group is carboxyl, the compound of formula (XIII) may be treated with a reagent capable of converting it to a corresponding activated acid in the presence of, or followed by reaction with, an appropriate reagent such as described above in respect of reactions with esters and acid chlorides.

Suitable reagents serving to effect formation of a corresponding activated acid may be sulphur oxychloride, a phosphorus halide such as phosphorus tri- or penta-chloride, a phosphorus oxyhalide such as the oxychloride, trifluoroacetic anhydride, an alkyl-(e.g. ethyl-) chloroformate, or any other suitable agent which will be apparent to those skilled in the art. A list of such reagents appears in Houben-Weyl (ref. (f) <u>supra</u>), Part 1, p.29. Conveniently, the reaction may be performed in a suitable solvent such as toluene, desirably in the presence of an appropriate catalyst such as dimethylformamide. Alternatively the reaction may be effected by reaction with an appropriate weak or volatile base such as ammonia, preferably by heating. Such a reaction conveniently may be effected using a stream of ammonia or by use of ammonia <u>in situ</u> in the presence of a dehydrating agent.

In reactions (a) and (b) and elsewhere in this specification, unless expressly indicated to the contrary, the or any leaving group may be selected from any of those known in the art of chemistry, for example, as defined in J. March, Advanced Organic Chemistry, 3rd Edn., 1977, McGraw Hill, New York, p.187. Such leaving groups include halo (preferably chloro or bromo), mesyl (i.e. methanesulphonyloxy), tosyl (i.e. toluenesulphonyloxy) and triflate (i.e. trifluoromethanesulphonyloxy).

It will be appreciated that where any process according to the present invention does not inherently result in preparation of a desired individual isomer of a compound of formula (I) or a physiologically acceptable salt thereof, that process may involve the separation of isomers, either of the product itself or of any appropriate intermediate at any convenient stage of the reaction, for example, by means of column chromatography.

In reaction (a), the compound of formula (II) may be prepared according to the general method described in the aforementioned U.S. Patent 3,705,907. The compounds of formula (III) may be prepared by methods generally analogous to those described in the above-mentioned European Patent 0012 401 and also in European Patent 0126986 A1.

In reaction (b), compounds of formula (IV) wherein in the definition of A, Q is -CO- and k and m are both 1, may be prepared by reacting a compound of formula (XIV):

$$B\text{-}(X)\text{-}R^{13} \qquad \text{(XIV)}$$

with a compound of formula (XV):

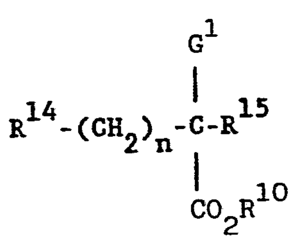

(XV)

wherein B, $G^1$ n, $R^{10}$, and (X) are as hereinbefore defined, or, as appropriate, protected forms thereof, $R^{13}$ is a carboxyl group or a derivative thereof, $R^{14}$ is an amino group or a derivative thereof, and $R^{15}$ is a suitably protected group, and treating the reaction product with a suitable agent or agents to deprotect $R^{15}$ or convert it to the desired $R^9$ group. For example, if $R^9$ is a leaving group in compound (IV), $R^{15}$ in compound (XV) may be a suitably protected hydroxy group, such as t-butoxy, which may be converted to the desired leaving group by treating the reaction product with a suitable reagent or agents, for example methanesulphonyl halide to give $R^9$ = mesyl. Similarly, if $R^9$ is an amino group or a derivative thereof in compound (IV), $R^{15}$ in compound (XV) is a suitably protected amino group.

Compounds of formula (IV) wherein in the definition of A, k and m are both zero, may be prepared by reacting a compound of formula (XVI):

$$B-(X)-(CH_2)_n-R^{16} \qquad (XVI)$$

with a compound of formula (XVII):

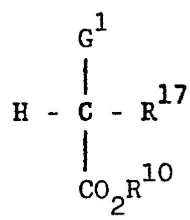

(XVII)

wherein B,$G^1$, n, $R^9$, $R^{10}$, and (X) are as hereinbefore defined, or, as appropriate, protected forms thereof, $R^{16}$ is a leaving group, and $R^{17}$ is a suitably protected amino group, treating the reaction product with a suitable agent or agents to deprotect $R^{17}$, and treating the deprotected product with a suitable agent or agents to either alkylate the amino group to give a compound (IV) in which $R^9$ = $NHQ^1$, wherein $Q^1$ is as hereinbefore defined, or to convert the amino group to a leaving group to give a compound (IV) in which $R^9$ is a leaving group. For example, when $R^{17}$ is PhCH=N-, where Ph = phenyl, it may be deprotected in the reaction product by treatment with a suitable base. Optional treatment of the product to convert the amino group to a leaving group may, for example, be effected

according to the general method described in E.D. Thorset, Tet. Lett., 1982, 23, 1875-6.

The compounds of formula (IV) in which in the definition of A, k and m are both zero, may also be prepared according to any of the methods described in Chapter 8 (pp. 246 ff) of the G.C. Barrett (Ed.) reference (designated m) *supra*).

The compounds of formula (V) are simple dipeptides or trivial derivatives thereof and may be prepared by methods well known to those skilled in the art.

In reaction (c), compounds of formula (VI) wherein, in the definition of A, Q is -CO- and k and m are both 1, may be prepared by reacting a compound of formula (XVI) as hereinbefore defined with a compound of formula (XVIII):

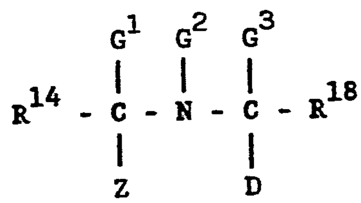

(XVIII)

wherein D, $G^1$ to $G^3$, $R^{14}$, and Z are as hereinbefore defined, or, as appropriate, protected forms thereof, and $R^{18}$ is a protected carboxyl group, and treating the reaction product to deprotect $R^{18}$.

Compounds of formula (VI) wherein in the definition of A, k and m are both zero, may be prepared by reacting a compound of formula (IV) as hereinbefore defined with a compound of formula (XIX):

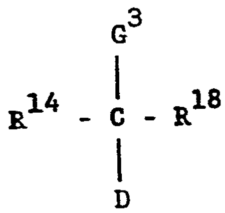

(XIX)

wherein D, $G^3$, $R^{14}$, and $R^{18}$ are as hereinbefore defined, or, as appropriate, protected forms thereof, and treating the reaction product to deprotect $R^{18}$.

The compounds of formula (VII) are simple amino acids or trivial derivatives thereof and may be prepared by methods well known to those skilled in the art.

In reaction (B) of optional reaction (i), the compounds of formula (VIII) to (X) wherein, in the definition of A, Q is -CO- and k and m are both 1, may be prepared by reaction of a compound of formula (XIV) as hereinbefore defined, with a compound of formula (XX), (XXI), or (XXII) as appropriate:

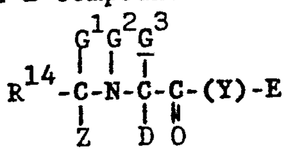

(XX)

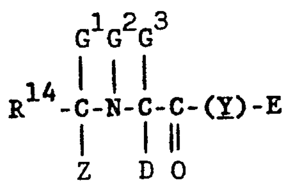

(XXI)

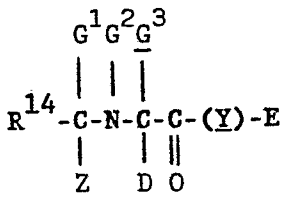

(XXII)

wherein D, E, $G^1$ to $G^3$, $\underline{G}^3$, $R^{14}$, (Y), and ($\underline{Y}$) are as hereinbefore defined, or, as appropriate, protected forms thereof. The reaction is preferably carried out under DCCI/HOBT conditions (<u>vide supra</u>) or by mixed anhydride coupling.

Compounds of formulae (VIII) to (X) wherein in the definition of A, k and m are both zero, may be prepared by reacting a compound of formula (IV) as hereinbefore defined ($R^9$ = leaving group) with a compound of formula (XXIII), (XXIV), or (XXV), as appropriate:

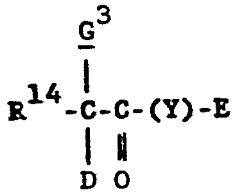

(XXIII)

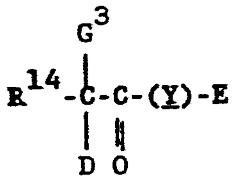

(XXIV)

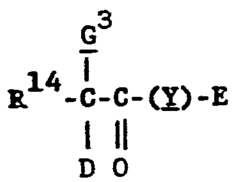

(XXV)

wherein D,E, $G^3$, $\underline{G}^3$, $R^{14}$, (Y), and (<u>Y</u>) are as hereinbefore defined, or, as appropriate, protected forms thereof.

In reaction (C) of optional reaction (i), the compounds of formula (XI) and (XII) may be prepared by reacting a compound of formula (XXVI):

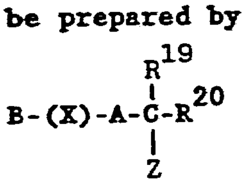

(XXVI)

wherein A,B,$G^1$, (X), and Z are as hereinbefore defined and $R^{19}$ and $R^{20}$ are either (i) $G^1$ and $R^{14}$ as hereinbefore defined or (ii) together form =O, with a compound of formula (XXVII):

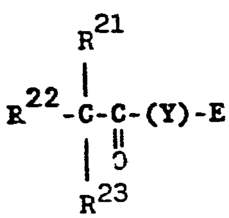

(XXVII)

wherein E and (Y) are as hereinbefore defined and, in case (i) referred to in the definition of compound (XXVI), $R^{21}$ is D as hereinbefore defined and $R^{22}$ and $R^{23}$ together form =O and in case (ii), $R^{21}$ to $R^{23}$ are D, $G^3$, and $R^{14}$ as hereinbefore defined or $R^{21}$ is $R^{14}$ as hereinbefore defined and $R^{22}$ and $R^{23}$ together form $=D^1$ as hereinbefore defined. These reactions are preferably carried out in a water-immiscible solvent such as toluene, benzene, or chloroform in the presence of an appropriate acid catalyst such as p-toluenesulphonic acid.

In reaction (D) of optional reaction (i), the compounds of formula (XIII) wherein $Z^1$ is cyano may be prepared by reacting a compound of formula (V) as hereinbefore defined with a compound of formula (XXVIII):

B-(X)-A-CHO (XXVIII)

wherein A, B, and (X) are as hereinbefore defined, in the presence of hydrogen cyanide, preferably formed in situ, for example, by mixing the reactants (V) and (XXVIII) in the presence of an alkali metal cyanide, such as sodium or potassium cyanide, and a suitable acid, such as acetic acid, preferably in an appropriate solvent such as methanol.

This reaction and the preparation of the starting materials (V) and (XXVIII) may follow the general methodology described in J. Med. Chem. 28, 432 (1985).

For a better understanding of the invention the following examples are given by way of illustration. In these examples and throughout the specification, all temperatures are in degrees Celsius unless othrwise indicated.

## Example 1

N-(1-Carboxy-5-[4-(2-hydroxy-3-isopropylaminopropoxy)benzylcarboxamido]-pentyl) alanylproline bistrifluoroacetate

(a) Methyl 4-(2-hydroxy-3-isopropylaminopropoxy)phenylacetate

Methyl 4-hydroxyphenylacetate (16.6g) was dissolved in 1N NaOH (100ml) and dioxane (20ml). Epichlorohydrin (23.1g, 2.5 equiv.) was added and the mixture was stirred at 80$^{o}$C for 3 hours. The mixture was cooled and extracted with 5:1 $CHCl_3$:dioxane (3 x 100ml). The combined organic extracts were dried ($MgSO_4$) and evaporated in vacuo. The residue was dissolved in a mixture of dioxane (60ml) and

isopropylamine (60ml) and heated at 65-70°C for 1 hour. The solution was evaporated <u>in vacuo</u> and the residue was extracted with 10% citric acid (5 x 50ml). The combined acid extracts were washed with ethyl acetate and then made basic with sodium carbonate.

(b) <u>Methyl 4-(2-hydroxy-3-isopropyl-t-butoxycarbonylaminopropoxy)phenyl acetate</u>

To a solution of the ester from step (a) (6.9g) in DMF (25ml) was added $BOC_2O$ (5.9g). After standing overnight, water (150ml) was added and the mixture extracted with ethyl acetate. The organic layer was washed 5 x with water, dried ($MgSO_4$), and evaporated.

(c) The crude BOC-protected ester from step (b) (10g) was mixed with SVM (920ml, 1.6 equiv.) and 2N NaOH (20ml) and the mixture heated on a steam bath for 30 minutes. The mixture was evaporated <u>in vacuo</u> and the residue dissolved in water and carefully acidified with dilute HCl. The oil which separated out was washed several times with water by decantation. After standing for 16 hours, the oil began to solidify. The solid was washed with water, dried, and recrystallised from ethyl acetate containing a little petroleum ether (b.p. 60-80°C).

(d) N-(1-<u>t</u>-Butoxycarbonyl-5-aminopentyl)alanylproline <u>t</u>-butyl ester (580g) and the acid from step (c) (500mg) were dissolved in DMF (8ml) and $Et_3N$ (0.14g, 1.0 equiv.) was added. The mixture was cooled on an ice bath, then stirred during the addition of 1-hydroxybenzotriazole (HOBT) (0.37g, 2.0 equiv.) followed by DCCI (0.31g, 1.1 equiv.). After stirring for 3 hours, the mixture was stored in a refrigerator. One week later, the mixture was filtered and the residue evaporated <u>in vacuo</u>, followed by the addition of ethyl acetate and washing with water, satd. aqu. $NaHCO_3$, 10% citric acid, water, and brine. The aqueous phases were extracted with ethyl acetate and the combined organic fractions dried ($MgSO_4$), followed by evaporation <u>in vacuo</u> to leave the product.

(e) The compound from step (d) (540mg) and anisole were mixed and stored in a refrigerator. A mixture of TFA (50ml), water (6.5ml) and anisole (5.5ml) were also cooled and, after about 45 minutes, the reagents were mixed and left to stand for 1 week. The mixture was then evaporated down and ether added, followed by re-treatment with more of the TFA mixture. The TFA was then removed in vacuo at ambient temperature, ether was added, and the mixture was stirred well to yield a white powder which after decanting-off of the supernatant was washed three more times in ether, followed by drying in vacuo to yield a colourless powder.

Calcd: C, 48.48; H, 5.849; N, 7.068
Found: C, 47.91; H, 5.791; N, 6.753

Examples 2-4

The following compounds in accordance with the invention were prepared in a manner analogous to the method of Example 1:

2. N-(1(S)-Carboxy-5-(2-[5-(2(S,R)-hydroxy-3-isopropylaminopropoxy)-naphth-1-yloxy]acetamido)pentyl)-(S,R)-alanyl-(S)-proline dihydrochloride acetate hemihydrate

   Calcd: (for 2HCl, AcOH, $0.5H_2O$) C, 52.84; H, 6.91; N, 7.25; Cl, 9.17
   Found: C, 52.60; H, 6.88; N, 7.45; Cl, 9.10

3. N-(1(S,R)-Carboxy-5-[5-(2(S,R)-hydroxy-3-isopropylaminopropoxy)carbazole- 3-carboxamido]pentyl)-(S,R)-alanyl-(S)-proline

4. N-(1(S,R)-Carboxy-5-[5-(2(S,R)-hydroxy-3-isopropylaminopropoxy)carbazole- 2-carboxamido]pentyl)-(S,R)-alanyl-(S)-proline

Example 5

N-(1(S)-Carboxy-5-[8-(2(S,R)-hydroxy-3-isopropylaminopropoxy)naphth-2-ylmethylamino]pentyl)-(S,R)-alanyl-(S)-proline tristrifluoroacetate

(a) Methyl-1-hydroxy-7-naphthoate

To a hydrogen chloride saturated solution of methanol (50ml) was added 1- hydroxy-7-naphthoic acid (5.0g) and the resultant mixture was heated at reflux for 2.5 hours, then concentrated to dryness to afford an off-white solid. This was dissolved in ethyl acetate (100ml) and washed in succession with saturated aqueous sodium bicarbonate (30ml), water (30ml) and then brine (30ml). The organic phase was dried ($MgSO_4$), filtered and concentrated to dryness to afford an off-white solid (5.36g; 99.7% yield) which was homogeneous on TLC (silica,ethyl acetate, uv/iodine), Rf 0.38.

(b) 2,3-Epoxypropoxy-7-naphthoic acid methyl ester

The naphthol from step (a) (2.02g, 0.01 moles) was dissolved in DMF (20ml) and cooled to 5°C under a nitrogen atmosphere. To this was added potassium t-butoxide (1.12g, 0.01 moles) at such a rate that the reaction mixture temperature did not exceed 15°C. After complete addition, the mixture was heated to 55°C for 1 hour and then epibromohydrin (1.37g, 0.01 moles) was added dropwise. The mixture was heated to 100°C for 2 hours, concentrated to semi-dryness, diluted with water and extracted into ether. The ethereal extract was washed with water, then brine, and dried ($MgSO_4$). Filtration and concentration afforded a viscous amber oil (2.48g, 96.1% yield). Homogeneous on TLC (silica, n-hexane:ether 1:1, uv/iodine), Rf 0.48.

(c) 1(S,R)-(2-Hydroxy-3-isopropylaminopropoxy)-7-naphthoic acid methyl ester

A mixture of the epoxide from step (b) (15.50g, 0.0213 moles), isopropylamine (100mls) and 1,4-dioxane (75mls) was heated at reflux for 3 days and then concentrated to dryness to afford an off-white solid. This was converted to the perchlorate salt to afford a white solid (6.70g, 75.4%). The free base was found to contain two products, as judged by TLC, which were inseparable by either chromatography of the free base or by recrystallisations of the perchlorate salt. This material was used without purification in the next step.

(d) 1-[1-(3-t-Butoxycarbonyl-3-isopropylamino-2(S,R)-hydroxypropoxy)]-7-naphthoic acid methyl ester

A mixture of the amine from step (c), as the perchlorate salt, (6.70g, 0.0161moles), sodium hydroxide (0.65, 0.0162 moles) and di-t-butyldicarbonate (3.52g, 0.0161 moles) in DMF (35mls) was stirred at room temperature for 20 hours, then concentrated to semi-dryness. The residue was diluted with water, extracted into ethyl acetate and the organic phase washed with water, dried ($MgSO_4$), filtered and concentrated to afford a viscous oil (7.00g, 104%) which solidified on standing m.p.107-109$^oC$. $C_{24}H_{34}N_1O_6$ requires C,66.65%; H,7.92%; N,3.25%; Found: C,66.25%; H,7.61%; N,3.29%.

(e) 1-[1-(3-t-Butoxycarbonyl-3-isopropylamino-2(S,R)-t-butyldimethyl silyloxy)propoxy]-7-naphthoic acid methyl ester

To a stirring mixture of the alcohol from step (d) (6.70g, 0.0160 moles), 4-dimethylaminopyridine (0.10g, 0.8 mmoles), and di-isopropylethylamine (2.10g, 0.0163 moles) in DMF (30ml) was added t-butyldimethylsilyl chloride (5.50g, 0.0365 moles) in one portion. The resultant mixture was stirred for 3 days at room temperature, poured into water (300 ml)/saturated sodium bicarbonate solution

(300ml) and extracted into ethyl acetate. The ethyl acetate solution was washed with water, then brine, and dried ($MgSO_4$). Filtration and concentration afforded a mobile colourless liquid which was purified by column chromatography (silica, 9:1 n-hexane:ethyl acetate) to afford a colourless oil (7.36g, 89.5% yield) which crystallised on standing to afford a white solid, m.p. 102°C. $C_{29}H_{45}N_1O_6Si$ requires C,65.50%; H,8.52%; N,2.63%; Found:C,65.4%; H,8.75%; N,2.59.

(f) 1-[1-(3-t-Butoxycarbonyl-3-isopropylamino-2(S,R)-t-butyldimethyl silyloxy)propoxy]-7-naphthoic acid

A solution of the methyl ester from step (e) (6.80g, 0.0128 moles) in methanol (50mls) was heated at reflux with sodium hydroxide (2.00g, 0.05 moles) for 18 hours. The RM was concentrated to semi-dryness, diluted with water and taken to pH 3.0 with solid citric acid. The mixture was then extracted into ethyl acetate which was washed with water, then brine, and dried ($MgSO_4$). Filtration and concentration afforded an off-white solid (5.86g, 88.5% yield), m.p. softens at about 167°C, collapses at 196°C. $C_{28}H_{43}NO_6Si$ requires C, 64.96%; H, 8.37%; N, 2.70%; Found: C,65.37%, H, 7.98%; N, 2.94%.

(g) 1-[1-(3-t-Butoxycarbonyl-3-isopropylamino-2(S,R)-t-butyldimethyl silyloxy)propoxy]-7-hydroxymethylnaphthalene

To a solution of the acid from step (f) (5.32g, 0.0103 moles) in THF (30ml) at 5°C and under static dry nitrogen was added a solution of borane-THF complex (18mls of 1 molar solution) at such a rate that the temperature of the mixture did not exceed 10°C. After complete addition, the reaction mixture was stirred with cooling for 30 minutes, then at room temperature for 2 hours. Water was then added, followed by the addition of saturated aqueous sodium bicarbonate solution (30mls), and the mixture was concentrated to semi-dryness. The residue was extracted into ether, which after washing with water and brine, was filtered and concentrated to afford the crude alcohol as a colourless gum (4.90g, 94.7% yield). Silica chromatography, eluting with 7:3 n-hexane:ethyl acetate, afforded pure alcohol (3.53g,

68% yield), m.p.81-82°C. $C_{28}H_{45}N_1O_5Si_1$ requires C,66.76%; H,9.00%; N,2.78%; Found: C,67.20%; H,8.90%; N,2.73%.

(h) 1-[1-(3-t-Butoxycarbonyl-3-isopropylamino-2(S,R)-t-butyldimethyl silyloxy)propoxy]-7-chloromethylnaphthalene

The alcohol from step (g) (3.32g, 6.6 mmoles) was dissolved in methylene chloride (50ml) and triethylamine (1.10ml, 7.90 mmoles). To this was added mesyl chloride (0.62ml, 8.0 mmoles) and the mixture was heated at reflux for 2 hours. TLC indicated the presence of starting material, so more triethylamine (0.5mml, 3.6 mmoles) and mesyl chloride (0.3 mls, 3.9 mmoles) was added and the mixture again heated at reflux for 1.5 hours. Pure chloride was obtained by silica chromatography eluting with 9:1 n-hexane:ethyl acetate to afford a colourless viscous oil (2.63g, 76% yield).

(i) N-(1(S)-Carboxy-t-butyl ester-5-[1-(3-t-butoxycarbonyl-3-isopropyl-amino-2-(S,R)-t-butyldimethylsilyloxy)propoxy]naphth-7-ylmethylamino)-(S,R)-alanyl-(S)-proline t-butyl ester

A mixture of N-(1(S)-t-butoxycarbonyl-5-aminopentyl)-(S,R)-alanyl-(S)-proline-t-butyl ester (2.43g, 5.69 mmoles), 2-chloromethylnaphthalene (9) (1.50g, 2.87 mmoles) and sodium bicarbonate (0.55g, 6.55 mmoles) in acetonitrile (30ml) was stirred at room temperature for 24 hours, then at 75°C for 20 hours. The reaction mixture was concentrated to near dryness, diluted with water and extracted into ethyl acetate. The extract was washed with water, then brine, and dried ($MgSO_4$). Filtration and concentration afforded a viscous oil (3.40g) which on TLC (silica, 80:20:1 $CHCl_3$:MeOH:$NH_4OH$), was shown to be a mixture of starting materials and product. The wanted material was obtained by column chromatography (silica) eluting with n-hexane, 1:1 n-hexane:ether, ethyl acetate, and finally 20% methanol in ethyl acetate to afford a near colourless gum (1.16g, 44% yield) as a mixture of diastereoisomers.

(j) N-(1(S)-Carboxy-5-[8-(2(S,R)-hydroxy-3-isopropylaminopropoxy)naphth -2-ylmethylamino]pentyl)-(S,R)-alanyl-(S)-proline tristrifluoroacetate

The protected compound from step (i) (0.48g, 0.526mmoles) in water (0.5ml) and trifluoroacetic acid (4.5ml) was stirred at room temperature for 20 hours, then concentrated to semi-dryness. The residue was dissolved in water and washed with ether, then the aqueous phase was concentrated to dryness and dried under high vacuum over phosphorus pentoxide to afford a light, sandy coloured solid (0.40g, 82% yield), m.p. softens at about 120°C. $C_{37}H_{48}O_{13}N_4F_9$ requires C, 47.90%; H, 5.20%; N,6.03%; Found:C,47.90%; H,5.70%; N,6.08%.

N-(1-t-Butoxycarbonyl-5-aminopentyl)alanylproline t-butyl ester, as used in step (d) of Examples 1 to 4, and chiral N-(1(S)-t-butoxycarbonyl-5-aminopentyl)-(S,R)-alanyl-(S)-proline t-butyl ester, as used in step (i) of Example 5, may be prepared by the following method using starting materials of the appropriate chirality:

(a) N-(2-bromopropionyl)proline t-butyl ester

A solution of proline t-butyl ester (1.71g) and triethylamine (1.08g) in dry dichloromethane (10ml) was cooled to -20°C and a solution of 2-bromopropionyl bromide (2.16g) in dichloromethane (5ml) at -20°C was then added in one portion to the stirred solution. The mixture was stirred for 30 minutes at -20°C, then allowed to warm to room temperature. Sufficient ethyl acetate was added to give two layers and the upper organic layer was washed successively with 10% citric acid, saturated sodium bicarbonate solution, water and lastly saturated brine. The solution was dried ($MgSO_4$) and evaporated in vacuo to give 2.8g of product as an oil. The n.m.r. spectrum in $CDCl_3$ shows the methyl protons at 1.5δ, the butyl protons at 1.8δ and the proline protons at 2.0, 3.66 and 4.44δ.

(b) N-(1-t-Butoxycarbonyl-5-aminopentyl)alanylproline t-butyl ester

Powdered sodium bicarbonate (0.93g) was added to a solution of t-butyl $N^{\varepsilon}$-benzyloxycarbonyl lysinate (3.36g) and N-(2-bromo propionyl)proline t-butyl ester from step (a) (3.08g) in dry acetonitrile (50ml). The mixture was refluxed gently for 24 hours. After cooling, acetonitrile was removed in vacuo and the residue was partitioned between water and ethyl acetate. The organic layer was washed with water, 10% citric acid, saturated sodium bicarbonate solution, water and saturated brine. The solution was dried ($MgSO_4$) and evaporated to give 3.88g of crude product. This was purified by column chromatography on silica gel (Merck 5734). Elution with 1:1 EtOAc:hexane gave 3.42g of colourless solid. The n.m.r. and mass spectra were consistent with N-(1-t-butoxycarbonyl-5-benzyloxycarbonyl aminopentyl)alanylproline t-butyl ester. 4.62g of the above benzyl compound was deprotected by hydrogenation in 1:1 EtOAc:MeOH (100ml) over 10% Pd/C catalyst (0.53g). Hydrogenation was complete after 4 hours. The mixture was filtered and the filtrate was evaporated to dryness in vacuo. The residue was treated with triethylamine (5ml) and ethyl acetate (20ml) was added. The precipitate was filtered off and the solvent was removed in vacuo to give 3.5g of the required N-(1-t-butoxycarbonyl-5-aminopentyl)alanylproline t-butyl ester. The n.m.r. spectrum in $CDCl_3$ was consistent with the structure.

Starting materials and reagents employed in the preparation of the compounds of Examples 1 to 5 are available commercially or may be obtained by methods described in the chemical literature.

## Pharmaceutical Formulations

In the following formulation examples, the "active compound" may be any compound of formula (I) or a physiologically acceptable salt thereof.

Example A:

TABLET

| | mg |
|---|---|
| Active Compound | 500 |
| Microcrystalline Cellulose | 100 |
| Polyvinylpyrrolidone | 10 |
| Sodium Starch Glycollate | 30 |
| Magnesium Stearate | 5 |
| | 645 |

Mix the active compound, the microcrystalline cellulose and the sodium starch glycollate. Granulate with the polyvinylpyrrolidone dissolved in alcohol. Dry. Mix in the magnesium stearate and compress (645 mg).

Example B:

CAPSULE

| | mg |
|---|---|
| Active Compound | 250 |
| Lactose | 150 |
| Starch | 25 |
| Magnesium Stearate | 5 |
| | 430 |

Mix the active compound, the lactose, the starch and the magnesium stearate. Fill into hard gelatin capsules.

Example C:

SUPPOSITORY

| | mg |
|---|---|
| Active Compound | 500 |
| PEG 400 | 800 |
| PEG 6000 | 1700 |
| | 3000 |

WMD/OLM/29th January 1987

Melt the PEG 6000. Mix in the PEG 400 followed by the active compound. Pour into a suppository mould and allow to set.

Example D:

I/V INJECTION

| | | |
|---|---|---|
| Active Compound | | 500mg |
| Water for Injection B.P. | to | 5.0ml |

Dissolve the active compound in about nine-tenths of the total volume of water for injection. When dissolution is complete, make up to volume with remaining water for injection.

Under aseptic conditions, sterilise the solution by filtration through a sterile, sterilising grade filter and pack the solution into previously sterilised ampoules, flushing the ampoules with nitrogen before and after filling.

Biological Examples

(a) β-Blockade in vitro

In an assay for β-adrenoceptor blockade in guinea pig left atria, a simple competitive blockade of responses to isoprenaline gave the following results:

| Compound of Example 2 | Compound of Example 5 |
|---|---|
| $pK_B$ 5.3 | $pK_B$ 7.2 |

(b) ACE inhibition in vitro

In any assay for angiotension converting enzyme inhibition in guinea pig ileum, dose-dependent inhibition of contractile responses to Angiotensin I gave the following results:

| Compound of Example 2 | Compound of Example 5 |
| --- | --- |
| $EC_{50}$ 13.5nM | $EC_{50}$ 9.6nM |

WMD/OLM/29th January 1987

Claims

1. A compound of formula (I)

```
                 G1G2G3
                 | | |
B-(X)-A-C-N-C-C-(Y)-E              (I)
        |   | ||
        Z   D O
```

wherein:

A is a group of formula $-Q_k-(NQ^1)_m-(CH_2)_n-$, where k and m are independently selected from 0 and 1, provided that m can only be 0 when k is 0, n is from 1 to 6, $Q^1$ is hydrogen or $C_{1-4}$ alkyl, and Q is selected from -CO-, $-CH_2-$, $-CH_2CO-$ and $-OCH_2CO-$, and any of the $-(CH_2)_n-$ groups are independently optionally substituted by one or two $C_{1-4}$ alkyl groups;

B is group of formula $-R^1-C(Q^2)(OH)-C(Q^3)(Q^4)-NQ^5-R^2$, where $R^1$ is a bond or $-OC(Q^6)(Q^7)-$, $R^2$ is hydrogen or $C_{1-6}$ alkyl, and $Q^2$-$Q^7$ are independently hydrogen, $C_{1-4}$ alkyl, or a group of formula $-(C(Q^8)(Q^9))_xPh$, in which x is 1 or 2, Ph is a phenyl group optionally substituted by one or more groups independently selected from hydroxy, $C_{1-4}$ alkyl and $C_{1-4}$ alkoxy, and $Q^8$ and $Q^9$ are independently hydrogen or $C_{1-4}$ alkyl;

E and Z are independently selected from carboxyl and carboxyl derivatives;

D is hydrogen or a saturated or unsaturated $C_{1-6}$ aliphatic substituent optionally substituted by an amino group;

$G^1$, $G^2$ and $G^3$ are independently selected from hydrogen and $C_{1-4}$ alkyl;

(X) is a mono-, bi-, or tri-cyclic aromatic ring system optionally containing one or more heteroatoms; and

(Y) is a mono- or bi-cyclic nitrogen-containing ring system (attached to $-CO-C(D)(G^3)-$ by said nitrogen atom) optionally containing one or more

other heteroatoms and optionally substituted by one or more substituents independently selected from $C_{1-6}$ alkyl and aryl (e.g. phenyl), or (Y) is a nitrogen atom attached to E directly by a bond or indirectly by a $C_{1-4}$ alkylene moiety and also attached to a $C_{5-7}$ cycloalkyl group; in either case (Y) may optionally be linked by a $C_{3-5}$ alkylene bridge to $G^3$ or D to form a closed ring;

and physiologically acceptable salts thereof;

with the proviso that:

when, in the definition of A, k and m are both 0 or both 1 and Q is a -CO- group, and, in the definition of B, $R^1$ is a group of formula $-OC(Q^6)(Q^7)-$ as hereinbefore defined and $R^2$ is a $C_{1-6}$ alkyl group;

D is hydrogen or a $C_{1-6}$ alkyl group;

(X) is a benzene ring or a naphthyl or indolyl ring system, any of which is optionally substituted in any position by one or more substituents independently selected from $C_{1-4}$ alkyl (itself optionally substituted by one or more halogen atoms), $C_{1-4}$ alkoxy, halo, nitro, amino, carboxyl, $C_{1-4}$ alkoxycarbonyl and hydroxy,

then (Y) is not a pyrrolidinyl, oxazolidinyl, or thiazolidinyl ring, or a ring system selected from indolinyl, quinolinyl and tetrahydroquinolinyl.

2. A compound of formula (I) according to claim 1, or a physiologically acceptable salt thereof, wherein in the definition of A, $Q^1$ is hydrogen and the $-(CH_2)_n-$ groups are not substituted by any $C_{1-4}$ alkyl group, in the definition of B, $Q^2$ to $Q^9$ are hydrogen and $R^2$ is a $C_{1-4}$ alkyl group, $G^1$ to $G^3$ are hydrogen, (X) is unsubstituted, and (Y) is a pyrrolidinyl, oxazolidinyl, or thiazolidinyl ring, or a tetraquinolinyl ring system.

3. A compound of formula (I) according to claim 2, or a physiologically acceptable salt thereof, wherein D is $C_{1-4}$ alkyl, E and Z are

carboxyl, (X) is an unsubstituted benzene ring or an unsubstituted naphthyl, indolyl, quinolyl, or carbazyl ring system, and (Y) is a pyrrolidinyl ring.

4. The compound
N-(1(S)-carboxy-5-[8-(2(S,R)-hydroxy-3-isopropylaminopropoxy)naphth-2--ylmethylamino]pentyl-(S,R)-alanyl-(S)-proline,

N-(1(S)-carboxy-5-[4-(2(S,R)-hydroxy-3-isopropylaminopropoxy)benzylcarboxamido]pentyl-(S,R)-alanyl-(S)-proline, or

N-(1(S)-carboxy-5-(2-[5-(2(S,R)-hydroxy-3-isopropylaminopropoxy)naphth-1-yloxy]acetamido)pentyl]-(S,R)-alanyl-(S)-proline,

or a half ester (as defined herein) of any thereof,

or a physiologically acceptable salt of any such compound.

5. A compound of formula (I) as claimed in claim 1, or a physiologically acceptable salt thereof, for use in medical therapy.

6. A compound of formula (I) according to claim 1, or a physiologically acceptable salt thereof, for use in the prophylaxis or treatment of a cardiovascular disorder, hyperaldosteronism, glaucoma, or migraine.

7. A compound of formula (I) according to claim 1, or a physiologically acceptable salt thereof, for use in the prophylaxis or treatment of a cardiovascular disorder which is hypertension, congestive heart failure, myocardial infarction, or ischaemic heart disease.

8. A compound of formula (I) according to claim 1, or a physiologically acceptable salt thereof, for use in the prophylaxis or treatment of an ischaemic heart disease which is angina pectoris.

WMD/OLM/29th January 1987

9. A compound of formula (I) according to claim 1, or a physiologically acceptable salt thereof, for use in the prophylaxis or treatment of a disease or disease symptom as defined in any of claims 6 to 8, which compound or salt is

N-(1(S)-carboxy-5-[8-(2(S,R)-hydroxy-3-isopropylaminopropoxy)naphth-2--ylmethylamino]pentyl-(S,R)-alanyl-(S)-proline,

N-(1(S)-carboxy-5-[4-(2(S,R)-hydroxy-3-isopropylaminopropoxy)benzylcarboxamido]pentyl-(S,R)-alanyl-(S)-proline, or

N-(1(S)-carboxy-5-(2-[5-(2(S,R)-hydroxy-3-isopropylaminopropoxy)naphth-1-yloxy]acetamido)pentyl]-(S,R)-alanyl-(S)-proline,

or a half ester (as defined herein) of any thereof,

or a physiologically acceptable salt of any such compound.

10. Use of a compound of formula (I) as claimed in claim 1, or a physiologically acceptable salt thereof, in the prophylaxis or treatment of a disease or disease symptom as defined in any of claims 6 to 8.

11. Use according to claim 10, wherein the compound of formula (I) or a physiologically acceptable salt thereof is

N-(1(S)-carboxy-5-[8-(2(S,R)-hydroxy-3-isopropylaminopropoxy)naphth-2--ylmethylamino]pentyl-(S,R)-alanyl-(S)-proline,

N-(1(S)-carboxy-5-[4-(2(S,R)-hydroxy-3-isopropylaminopropoxy)benzylcarboxamido]pentyl-(S,R)-alanyl-(S)-proline, or

N-(1(S)-carboxy-5-(2-[5-(2(S,R)-hydroxy-3-isopropylaminopropoxy)naphth-1-yloxy]acetamido)pentyl]-(S,R)-alanyl-(S)-proline,

or a half ester (as defined herein) of any thereof,

or a physiologically acceptable salt of any such compound.

12. A pharmaceutical formulation comprising a compound of formula (I) as claimed in claim 1, or a physiologically acceptable salt thereof, and an acceptable carrier therefor.

13. A process for the preparation of a compound of formula (I) as claimed in any of claims 1 to 4, or a physiologically acceptable salt thereof, which comprises coupling the aryl moiety (X), or any precursor thereto, the amino(hydroxy)alkoxy side chain B, or any precursor thereto, and the aminoacids or aminoacid derivatives, or any precursor thereto, which together form the compound of formula (I), or a precursor thereto, and, when appropriate, converting the precursor compound to the compound of formula (I) and, if desired, converting any such compound of formula (I) to another compound of formula (I) and, if desired, converting any compound of formula (I) so formed to a physiologically acceptable salt thereof.

Claims

1. A process for the preparation of a compound of formula (I)

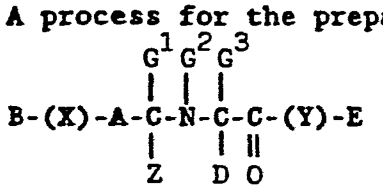

(I)

wherein:

A is a group of formula $-Q_k-(NQ^1)_m-(CH_2)_n-$, where k and m are independently selected from 0 and 1, provided that m can only be 0 when k is 0, n is from 1 to 6, $Q^1$ is hydrogen or $C_{1-4}$ alkyl, and Q is selected from $-CO-$, $-CH_2-$, $-CH_2CO-$ and $-OCH_2CO-$, and any of the $-(CH_2)_n-$ groups are independently optionally substituted by one or two $C_{1-4}$ alkyl groups;

B is group of formula $-R^1-C(Q^2)(OH)-C(Q^3)(Q^4)-NQ^5-R^2$, where $R^1$ is a bond or $-OC(Q^6)(Q^7)-$, $R^2$ is hydrogen or $C_{1-6}$ alkyl, and $Q^2$-$Q^7$ are independently hydrogen, $C_{1-4}$ alkyl, or a group of formula $-(C(Q^8)(Q^9))_xPh$, in which x is 1 or 2, Ph is a phenyl group optionally substituted by one or more groups independently selected from hydroxy, $C_{1-4}$ alkyl and $C_{1-4}$ alkoxy, and $Q^8$ and $Q^9$ are independently hydrogen or $C_{1-4}$ alkyl;

E and Z are independently selected from carboxyl and carboxyl derivatives;

D is hydrogen or a saturated or unsaturated $C_{1-6}$ aliphatic substituent optionally substituted by an amino group;

$G^1$, $G^2$ and $G^3$ are independently selected from hydrogen and $C_{1-4}$ alkyl;

(X) is a mono-, bi-, or tri-cyclic aromatic ring system optionally containing one or more heteroatoms; and

(Y) is a mono- or bi-cyclic nitrogen-containing ring system (attached to $-CO-C(D)(G^3)-$ by said nitrogen atom) optionally containing one or more

0237239

other heteroatoms and optionally substituted by one or more substituents independently selected from $C_{1-6}$ alkyl and aryl (e.g. phenyl), or (Y) is a nitrogen atom attached to E directly by a bond or indirectly by a $C_{1-4}$ alkylene moiety and also attached to a $C_{5-7}$ cycloalkyl group; in either case (Y) may optionally be linked by a $C_{3-5}$ alkylene bridge to $G^3$ or D to form a closed ring;

and physiologically acceptable salts thereof;

with the proviso that:

<u>when</u>, in the definition of A, k and m are both 0 or both 1 and Q is a -CO- group, and, in the definition of B, $R^1$ is a group of formula $-OC(Q^6)(Q^7)-$ as hereinbefore defined and $R^2$ is a $C_{1-6}$ alkyl group;

D is hydrogen or a $C_{1-6}$ alkyl group;

(X) is a benzene ring or a naphthyl or indolyl ring system, any of which is optionally substituted in any position by one or more substituents independently selected from $C_{1-4}$ alkyl (itself optionally substituted by one or more halogen atoms), $C_{1-4}$ alkoxy, halo, nitro, amino, carboxyl, $C_{1-4}$ alkoxycarbonyl and hydroxy,

<u>then</u> (Y) is not a pyrrolidinyl, oxazolidinyl, or thiazolidinyl ring, or a ring system selected from indolinyl, quinolinyl and tetrahydroquinolinyl;

the process comprising coupling the aryl moiety (X), or any precursor thereto, the amino(hydroxy)alkoxy side chain B, or any precursor thereto, and the aminoacids or aminoacid derivatives, or any precursor thereto, which together form the compound of formula (I), or a precursor thereto, and, when appropriate converting the precursor compound to the compound of formula (I) and, if desired, converting any such compound of formula (I) to another compound of formula (I) and, if desired, converting any compound of formula (I) so formed to a physiologically acceptable salt thereof.

WMD/OLM/29th January 1987

2. A process according to claim 1, which comprises reactng a compound $R_1$ with a compound $R_2$, wherein:

(a) $R_1$ is a compound of formula (II)

$$B\text{-}(X)\text{-}Q\text{-}R^7 \qquad (II)$$

and $R_2$ is a compound of formula (III)

```
                  G1G2G3
                  | | |
R8-(CH2)n-C-N-C-C-(Y)-E          (III)
                  |   | ||
                  Z   D O
```

;or

(b) $R_1$ is a compound of formula (IV)

```
                        G1
                        |
B-(X)-(CH2)n-C-R9                (IV)
                        |
                        CO2R10
```

and $R_2$ is a compound of formula (V)

```
        G3
        |
R11-C-C-(Y)-CO2R12               (V)
        | ||
        D O
```

;or

(c) $R_1$ is a compound of formula (VI)

```
                G1G2G3
                | | |
B-(X)-A-C-N-C-C-OH               (VI)
                |   | ||
                Z   D O
```

and $R_2$ is a compound of formula (VII)

$$H\text{-}(Y)\text{-}E \qquad (VII)$$

wherein the hydrogen H is bonded to the nitrogen atom of (Y):

wherein, in the compound of formula (II) to (VII), A, (B), D, E, $G^1$ to $G^3$, n, X, (Y) and Z are as hereinbefore defined, $R^7$, $R^8$, $R^9$ and $R^{11}$ are independently selected from an amino group, an appropriate derivative thereof, or a leaving group such that when $R^7$ and/or $R^9$ is an amino group

or an appropriate derivative thereof, $R^8$ and/or $R^{11}$ is a leaving group, or, conversely, when $R^8$ and/or $R^{11}$ is an amino group or an appropriate derivative thereof, $R^7$ and/or $R^9$ is a leaving group, and $R^{10}$ and $R^{12}$ are suitable carboxyl protecting groups;

and subsequently, if desired, performing one or more of the following optional reactions in any desired order:

(i) where the product of the reaction of compounds $R_1$ and $R_2$ is a precursor to a compound of formula (I), subjecting that precursor to such conditions and/or treating it with an appropriate agent or agents to bring about formation of a compound of formula (I);

(ii) converting any compound of formula (I) so formed to another compound of formula (I); and

(iii) converting any compound of formula (I) to a physiologically acceptable salt thereof.

3. A process according to claim 1 or 2, which comprises:

(A) for the preparation of compounds of formula (I) wherein k is 1 and Q is other than methylene, reacting a compound of formula (II) wherein B, Q, and (X) are as hereinbefore defined, except that Q is not methylene, and $R^7$ is a hydroxy group and/or together with Q forms a carboxyl group or a derivative thereof, with a compound of formula (III) wherein D, E, $G^1$ to $G^3$, n, and Z are as hereinbefore defined and $R^8$ is an amino group or derivative thereof;

(B) reducing a compound of formula (VIII), (IX), or (X):

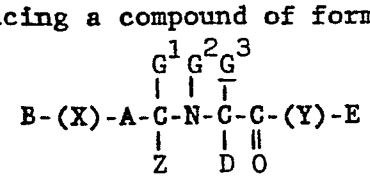

(VIII)

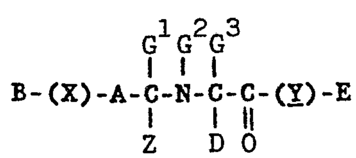

(IX)

WMD/OLM/29th January 1987

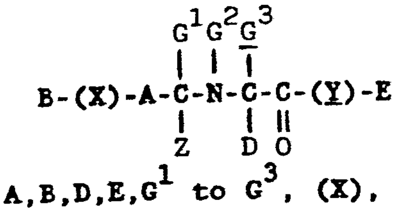 (X)

wherein A,B,D,E,$G^1$ to $G^3$, (X), (Y), and Z are as hereinbefore defined, or, as appropriate, protected forms thereof, and $\underline{G}^3$ and ($\underline{Y}$) are unsaturated forms of $G^3$ and (Y) respectively;

(C) for the preparation of compounds of formula (I) wherein $G^1$ and $G^2$ are hydrogen or $G^3$ is hydrogen, reducing a compound of formula (XI) or (XII) respectively:

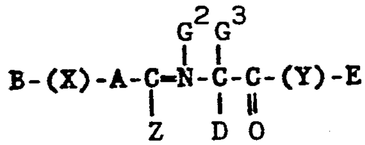 (XI)

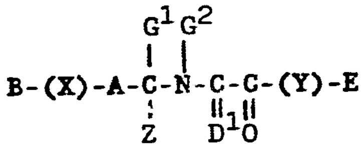 (XII)

wherein A,B,D,E,$G^1$ to $G^3$, (X), (Y), and Z are as hereinbefore defined, or as appropriate, protected forms thereof, and $D^1$ is a group analogous to D as hereinbefore defined (other than hydrogen) which lacks one hydrogen atom so that it is bonded to the carbon of the -N($G^2$)-C- moiety by a double bond;

(D) for the preparation of compounds of formula (I) wherein Z is a carboxyl or carbamoyl group by treating a compound of formula (XIII):

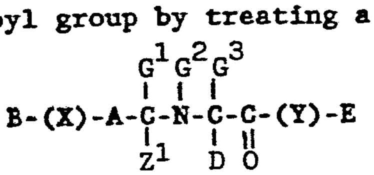 (XIII)

wherein A,B,D,E,$G^1$ to $G^3$, (X), and (Y) are as hereinbefore defined, or as appropriate, protected forms thereof, and $Z^1$ is an appropriate carboxyl or carbamoyl precursor group, with an agent or agents capable of converting the precursor group $Z^1$ to a carboxyl or carbamoyl group;

and, if desired, effecting one or more of the optional reactions (i) to (iii) defined in claim 2, or, as appropriate, one or more further of said optional reactions, in any desired order.

WMD/OLM/29th January 1987

4. A process according to claim 3(A), wherein the reaction is performed using dicyclohexylcarbodiimide (DDCI) in the presence of 1-hydroxybenzotriazole (HOBT) to suppress recemisation, in a suitable solvent at a temperature of from -15°C to room temperature.

5. A process according to claim 2(b), wherein the reaction is performed at room temperature or with heating as appropriate, in a suitable solvent.

6. A process according to claim 2(c), wherein the reaction is performed under the DDCI/HOBT conditions defined in claim 4, or by mixed anhydride coupling.

7. A process according to claim 3(B) or (C), wherein the reaction is performed by hydrogenation over a suitable catalyst.

8. A process according to claim 3(D), wherein the reaction comprises hydrolysis of a compound of formula (XIII) in which $Z^1$ represents cyano, or reaction of a compound of formula (XIII) in which $Z^1$ is an appropriate carbonyl derivative with one or more suitable agents serving to effect amination of said carbonyl derivative.

9. A process according to any of claims 1 to 8, wherein in the compound of formula (I) or a physiologically acceptable salt thereof so prepared, in the definition of A, $Q^1$ is hydrogen and the $-(CH_2)_n-$ groups are not substituted by any $C_{1-4}$ alkyl group, in the definition of B, $Q^2$ to $Q^9$ are hydrogen and $R^2$ is a $C_{1-4}$ alkyl group, $G^1$ to $G^3$ are hydrogen, (X) is unsubstituted, and (Y) is a pyrrolidinyl, oxazolidinyl, or thiazolidinyl ring, or a tetrahydroquinolinyl ring system.

10 A process according to claim 9, wherein in the compound of formula (I) or a physiologically acceptable salt thereof so prepared, D is $C_{1-4}$ alkyl, E and Z are carboxyl, (X) is an unsubstituted benzene ring or an unsubstituted naphthyl, indolyl, quinolyl, or carbazyl ring system, and (Y) is a pyrolidinyl ring.

WMD/OLM/29th January 1987

11. A process according to any of claims 1 to 10, wherein the compound of formula (I) or a physiologically acceptable salt thereof so prepared, is

N-(1(S)-carboxy-5-[8-(2(S,R)-hydroxy-3-isopropylaminopropoxy)naphth-2--ylmethylamino]pentyl-(S,R)-alanyl-(S)-proline,

N-(1(S)-carboxy-5-[4-(2(S,R)-hydroxy-3-isopropylaminopropoxy)benzylcarboxamido]pentyl)-(S,R)-alanyl-(S)-proline, or

N-(1(S)-carboxy-5-(2-[5-(2(S,R)-hydroxy-3-isopropylaminopropoxy)naphth-1-yloxyacetamido)pentyl]-(S,R)-alanyl-(S)-proline,

or a half ester (as defined herein) of any thereof,

or a physiologically acceptable salt of any such compound.

12. A process for the preparation of a pharmaceutical formulation by:

a) preparing a compound of formula (I), or a physiologically acceptable salt thereof, by a process according to any of claims 1 to 11; and

b) admixing the resulting product with an acceptable carrier therefor.

WMD/OLM/29th January 1987